# FASCICULE DE BREVET EUROPEEN

(11) **EP 2 666 421 B1**
(45) Date de publication et mention de la délivrance du brevet: **29.06.2016**
(21) Numéro de dépôt: 13168581.0
(22) Date de dépôt: 21.05.2013
(51) Int. Cl.: A61B 17/34, A61N 1/36, A61N 1/05

(54) **Dispositif d'assistance à la chirurgie otologique d'un patient à implanter avec un implant cochléaire**
Assistenzvorrichtung für die otologische Chirurgie bei einem Patienten, bei dem ein Cochlearimplantat eingesetzt werden soll
Device for assisting with otologic surgery of a patient to be implanted with a cochlear implant

(30) Priorité: 23.05.2012 FR 1254730
(43) Date de publication de la demande: 27.11.2013
(73) Titulaire: Collin, 92220 Bagneux (FR)
(72) Inventeur: Sterkers, Olivier, 75016 Paris (FR); Bozorg Grayeli, Alexis, 75017 Paris (FR); Miroir, Mathieu, 91300 Massy (FR); Nguyen, Yann, 75020 Paris (FR)
(74) Mandataire: Bonnet, Michel

(56) Documents cités:
- US-A1- 2010 114 288
- US-A1- 2011 319 913
- US-B1- 6 228 089

## Description

L'invention est relative à un dispositif d'assistance à la chirurgie otologique d'un patient à implanter avec un implant cochléaire.

### ARRIERE-PLAN TECHNOLOGIQUE DE L'INVENTION

Les surdités totales ou partielles sont maintenant traitées par mise en place dans la cochlée d'un implant cochléaire, plus précisément d'un porte-électrodes portant un faisceau d'électrodes reliées à un récepteur/stimulateur implanté dans le crâne du patient.

La technique classique d'implantation cochléaire consiste à pratiquer une mastoïdectomie par ablation de l'os mastoïdien se projetant en arrière du pavillon de l'oreille. Puis un passage étroit entre le nerf facial et le conduit auditif externe est dégagé au moyen d'une tympanotomie postérieure. Enfin, une cochléostomie donne accès à l'intérieur de la cochlée pour permettre la mise en place du porte-électrodes dans la cochlée. Cette technique est considérée comme très invasive.

Il a été proposé récemment d'effectuer ces opérations à l'aide d'un robot d'assistance chirurgicale, en guidant ce dernier au moyen d'algorithmes de navigation se basant sur des images préopératoires ou peropératoires obtenues par exemple par un scanner. En particulier, le robot comporte un bras dont la tête est munie d'un outil de forage (type fraise ou foret) pour creuser un passage d'accès à l'entrée de la cochlée au travers de l'os mastoïdien. Ces techniques sont nettement moins invasives.

Cependant, la précision d'une telle navigation (de l'ordre du millimètre) n'est pas suffisamment importante pour assurer un passage au travers de la mastoïde sans risque de blessure du nerf facial. Celui-ci est en effet situé sur le trajet de l'approche au niveau de la tympanotomie postérieure.

L'état de la technique est notamment illustré par les documents US 2011/319913 A1 et US 6 228 089 B1.

### OBJET DE L'INVENTION

L'invention a pour objet un dispositif d'assistance à la chirurgie otologique d'un patient à implanter avec un implant cochléaire, permettant la détermination d'une trajectoire d'accès à l'entrée d'une cochlée, qui, si elle est suivie par un outil de forage, doit permettre d'accéder à l'entrée de la cochlée tout en évitant toute lésion au nerf facial.

### BREVE DESCRIPTION DE L'INVENTION

En vue de la réalisation de ce but, on propose un dispositif d'assistance à la chirurgie otologique d'un patient à implanter avec un implant cochléaire, comportant :
- des moyens de repérage d'un point d'entrée sur la surface de la cochlée et d'un point de contrôle dans le sinus tympani, ces moyens étant opérables par le conduit auditif externe ;
- des moyens de traitement pour, à partir de coordonnées desdits points, déterminer une trajectoire rectiligne passant par ces deux points et définissant un point d'accostage sur la surface externe de l'os mastoïdien ;
- des moyens pour creuser un passage jusqu'en regard de la cochlée au travers de l'os mastoïdien en suivant ladite trajectoire rectiligne.

Selon un mode particulier de mise en oeuvre de l'invention, le repérage des points est effectué au moyen d'un palpeur monté à l'extrémité d'un bras de robot. Le praticien amène le palpeur au contact des points recherchés et les coordonnées de ces points sont relevées par simple lecture des signaux des capteurs de position du bras. Une précision importante peut couramment être atteinte avec les bras de robots actuellement développés.

De préférence, le point repéré formant le point d'ertrée dans la cochlée se situe sur la berge antérieure inférieure de la fenêtre ronde de la cochlée. De préférence également, le point repéré formant le point de contrôle dans le sinus tympani se situe en arrière du rebord du sulcus tympani.

Ces points permettent de définir sur la mastoïde un point d'accostage se trouvant sensiblement dans la zone cribriforme de celle-ci, parfaitement accessible à un outil de forage.

Diverses simulations ont permis de montrer qu'en forant l'os mastoïdien en suivant scrupuleusement la trajectoire rectiligne ainsi déterminée, on dégage un chemin d'accès jusqu'à la zone de cochléostomie qui évite le nerf facial. Ainsi, on parvient à ménager un accès à la cochlée, tout en minimisant les opérations invasives et en évitant de blesser le nerf facial.

Selon un mode particulier de mise en oeuvre de l'invention, l'outil de forage ayant servi à forer l'os mastoïdien sert également à réaliser la cochléostomie, dans le même mouvement. Le forage ainsi réalisé s'apparente alors à une tunnelisation dégageant un accès direct à la cochlée pour son traitement ou son appareillage au moyen d'un porte-électrodes.

### DESCRIPTION DES FIGURES

L'invention sera mieux comprise à la lumière de la description qui suit d'un mode particulier de mise en oeuvre de l'invention, en référence aux figures des dessins annexés, parmi lesquelles :
- la figure 1 est une vue schématique d'un dispositif d'assistance à la chirurgie otologique ;
- la figure 2 est une vue de côté partielle du crâne du patient, montrant les points repérés ;
- la figure 3 est une vue en coupe du crâne du patient, montrant également les points repérés.

### DESCRIPTION DETAILLEE D'UN MODE DE MISE EN OEUVRE DE L'INVENTION

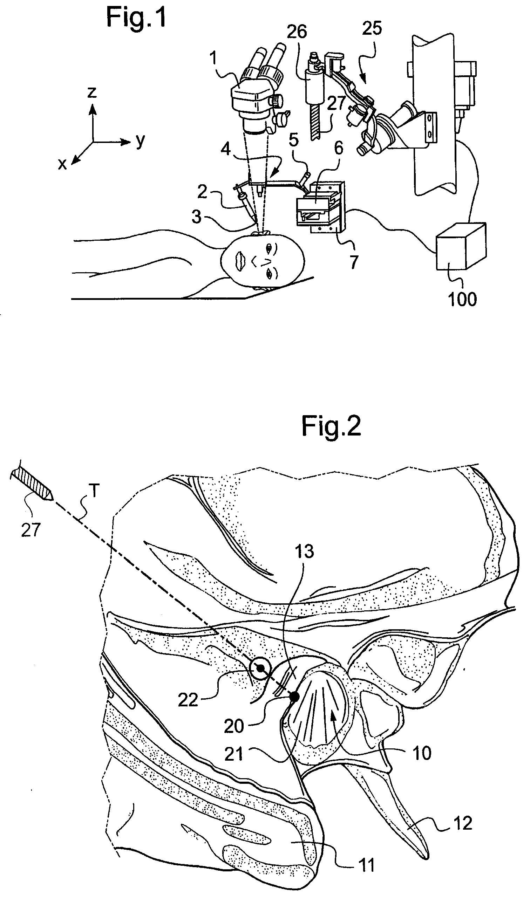

L'invention vise à ménager un accès à la cochlée de façon mini-invasive, en traumatisant le moins possible le patient. Comme cela est visible à la figure 1, le patient est en position allongée et sa tête est maintenue immobilisée sur le côté, de façon à ce que le conduit auditif externe s'étende vers le haut. Les moyens d'immobilisation sont bien connus et ne font pas partie de l'invention.

Le praticien dispose de moyens d'inspection visuelle du conduit auditif externe, en l'occurrence un microscope opératoire 1 dont le champ de vision est figuré en pointillés. Le praticien dispose par ailleurs d'un palpeur 2 ayant une extrémité sensible 3. Le palpeur 2 est ici monté à l'extrémité distale d'un bras articulé 4 dont chacune des articulations est munie d'un capteur de position angulaire 5 (l'un seul de ces capteurs est référencé). Le bras articulé 4 a une extrémité proximale reliée à une base 6 montée mobile sur un support 7 selon trois axes de translation. Chaque axe est équipé de capteurs de position correspondants. En exploitant les signaux des capteurs de position, il est possible de repérer à tout moment la position dans l'espace de la pointe sensible 3 du palpeur 2. Ici, les coordonnées des points ainsi repérées sont exprimées dans un repère absolu R. Les signaux des capteurs de position sont collectés par une unité de traitement 100 (par exemple un ordinateur personnel muni d'une carte d'acquisition adéquate) capable de calculer les coordonnées des points palpés en fonction des signaux des capteurs de position.

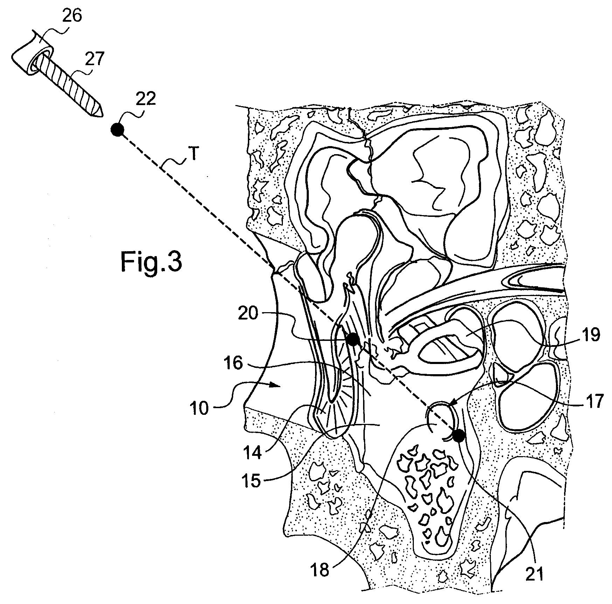

La façon dont le palpeur est utilisé est maintenant détaillé en référence à la figure 2, sur laquelle on reconnaît l'environnement crânien du conduit auditif externe 10, notamment le processus mastoïde 11, le processus styloïde 12, et l'épine supra-méatique de Henlé 13, mais également en référence à la figure 3, sur laquelle on reconnaît le conduit auditif externe 10, le tympan 14, le sinus tympani 15, le rebord 16 du sillon tympanique, la fenêtre ronde 17 de la cochlée et la berge inférieure antérieure 18 de cette dernière, et enfin le nerf facial 19, dont on observe qu'il passe à proximité de la zone de forage.

Après avoir décollé la peau du conduit auditif externe et le tympan, et pratiqué le cas échéant une tympanostomie, le praticien introduit le palpeur dans le conduit auditif externe 10 et amène la pointe sensible 3 du palpeur 2 dans la zone du sinus tympani, plus précisément à sensiblement un millimètre en arrière du rebord 16 du sulcus tympani. Le praticien appuie sur un bouton d'acquisition associé au palpeur 2 pour relever et mémoriser dans l'unité de traitement 100 les coordonnées du point ainsi palpé, que l'on appellera ici point de contrôle 20.

Puis le praticien enfonce plus avant le palpeur 2 pour aller palper un point sur la cochlée, situé plus précisément sur la berge inférieure et antérieure 18 de la fenêtre ronde de la cochlée. De la même façon, le praticien appuie sur un bouton d'acquisition associé au palpeur 2 pour relever et mémoriser dans l'unité de traitement 100 les coordonnées du point ainsi palpé, que l'on appellera ici point d'entrée 21. Le point d'entrée 21 est sensiblement au centre de la cochléostomie à venir.

Selon une variante de mise en oeuvre, l'acquisition des deux points est inversée se sorte à palper le point de contrôle 20 dans le sinus tympani avant de palper le point d'entrée 21 sur la surface de la cochlée.

Par ailleurs, et selon une autre variante de mise en oeuvre, la palpation est assistée ; à cet effet, si le bras articulé 4 est motorisé, le palpeur 2 est amené automatiquement à proximité des points à palper par l'unité de traitement 100 qui commande les moteurs du bras articulé, de sorte à guider le palpeur 2 en se basant par exemple sur des images préopératoires du conduit auditif externe. Il restera alors au praticien à venir palper très précisément les points recherchés en reprenant la main et en guidant le palpeur par exemple à l'aide d'une manette ou de tout autre organe de commande.

Selon l'invention, l'unité de traitement 100 est programmée pour, après avoir acquis le point de contrôle 20 et le point d'entrée 21, déterminer l'équation d'une trajectoire rectiligne T passant par ces deux points. C'est le long de cette trajectoire que sera ménagé le passage vers la cochlée. La trajectoire T définit un point sur la surface externe du crâne, dans la zone cribriforme de l'os mastoïdien, qui formera le point d'accostage 22 de l'outil avec l'os mastoïdien.

A cet effet, le dispositif de l'invention comprend un bras robot 25 articulé et motorisé qui est muni à son extrémité distale d'une broche motorisée 26 portant un outil de forage, en l'occurrence ici une fraise 27. Le bras robot 25 est connecté à l'unité de traitement 100 pour que celle-ci commande le bras robot 25 afin d'aligner l'axe de la fraise 27 sur la trajectoire rectiligne T, mettre en rotation celui-ci et procéder au forage de l'os mastoïdien selon la trajectoire rectiligne T.

La fraise 27 accoste alors le crâne au point d'accostage 22, creuse l'os mastoïdien en direction du point de contrôle 20, débouche dans le conduit auditif externe et continue son chemin vers le point d'entrée 21. Ce faisant, la fraise 27 dégage un passage jusqu'en regard de la cochlée. En continuant plus avant, la fraise 27 pratique dans la paroi de la cochlée une cochléostomie libérant ainsi un passage complet jusqu'à l'intérieur de la cochlée, permettant d'introduire dans celle-ci le porte-électrodes d'un implant cochléaire.

L'opération de forage se fait avantageusement sous surveillance optique au moyen du microscope opératoire 1. La surveillance optique est avantageusement complétée par une surveillance électromyographique du nerf facial permettant de détecter à tout moment un éventuel contact de l'outil avec le nerf facial, voire de connaître à tout moment la distance entre la pointe de l'outil et le nerf facial selon un procédé connu en soi.

L'invention n'est bien sûr pas limitée à ce qui vient d'être décrit, mais englobe toute variante entrant dans le cadre défini par les revendications.

En particulier, bien que dans le mode de mise en oeuvre illustré, le palpeur et l'outil de forage soient ici portés par des bras distincts, ils pourront être portés par le même bras, soit simultanément, soit en séquence. Le bras est alors de préférence un bras articulé, motorisé, et équipé de capteurs de positions à chacun de ses degrés de liberté. Par exemple, on pourra utiliser un dispositif de chirurgie assistée connu sous le nom de Robotol, présenté dans « Robotol : from design to évaluation of a robot for middle ear surgery » Miroir, M. & al, in Intelligent Robotos and Systems, 2010 IEEE/RSI International conference.

Bien que dans l'exemple illustré, les moyens de repérage des points d'entrée et de contrôle comprennent ici un palpeur, tous autres moyens de repérage pourront être utilisés du moment que ces moyens de repérage soient opérables par le conduit auditif externe, comme par exemple une illumination laser du point à repérer, la tache lumineuse ainsi créée étant localisée à l'aide d'une caméra CCD. On pourra également utiliser des méthodes de stéréovision ou des méthodes de repérage électromagnétique dans lesquelles un stylet portant des éléments de son positionnement dans l'espace est manipulé par le chirurgien.

On pourra encore mettre en oeuvre des méthodes de repérage en corrélation avec des images obtenues préalablement par scanner ou IRM.

Bien que dans le mode de mise en oeuvre illustré, le même outil de forage est utilisé pour réaliser la mastoïdostomie et la cochléostomie, on pourra réaliser le mastoïdostomie avec un premier outil, puis changer d'outil pour réaliser la cochléostomie.

## Revendications

1. Dispositif d'assistance à la chirurgie otologique d'un patient à implanter avec un implant cochléaire, comportant des moyens de forage (25, 27) pour forer un passage jusqu'en regard de la cochlée au travers de l'os mastoïdien, **caractérisé en ce qu'**il comporte en outre :
- des moyens de repérage d'un point d'entrée (21) sur la surface de la cochlée et d'un point de contrôle (20) dans le sinus tympani, ces moyens de repérage étant opérables par le conduit auditif externe ;
- des moyens de traitement (100) pour, à partir de coordonnées desdits points, déterminer une trajectoire rectiligne (T) passant par ces deux points et définissant un point d'accostage (22) sur la surface externe de l'os mastoïdien ;
- les moyens de forage étant adaptés à forer en suivant ladite trajectoire rectiligne, de sorte à créer un passage jusqu'à la cochlée.

2. Dispositif selon la revendication 1, dans lequel les moyens de repérage comportent un palpeur (2) ayant une pointe sensible (3) pouvant être amenée au contact de la cochlée et du sinus tympani par le conduit auditif externe pour palper le point d'entrée et le point de contrôle.

3. Dispositif selon la revendication 2, dans lequel le palpeur (2) est monté à l'extrémité d'un bras articulé (4) comportant des capteurs de position (5) associés à chacun de ses degrés de liberté, de sorte que la position de la pointe sensible du palpeur puisse être déduite par l'unité de traitement à partir des signaux générés par les capteurs de position.

4. Dispositif selon la revendication 1, dans lequel les moyens de forage comportent un bras articulé et motorisé portant à son extrémité un outil de forage (27), ledit bras étant commandable pour aligner et déplacer l'outil de forage selon ladite trajectoire (T).

5. Dispositif selon la revendication 3 et la revendication 4, dans lequel le palpeur et l'outil de forage sont portés par le même bras articulé, motorisé, et pourvu de capteurs de position associés à chacun de ses degrés de liberté.

6. Dispositif selon la revendication 1, comportant des moyens de surveillance optique (1) du conduit auditif externe.

7. Dispositif selon la revendication 1, comportant des moyens de surveillance électromyographique du nerf facial.

## Patentansprüche

1. Vorrichtung zur Unterstützung der otologischen Chirurgie bei einem Patienten, bei dem ein Cochlea-Implantat eingesetzt werden soll, wobei die Vorrichtung Bohrmittel (25, 27) zum Bohren eines Durchgangs bis zur Cochlea durch den Mastoidknochen umfasst, **dadurch gekennzeichnet, dass** sie außerdem Folgendes umfasst:
- Mittel zum Lokalisieren eines Eintrittspunkts (21) auf der Oberfläche der Cochlea und eines Kontrollpunkts (20) im Sinus tympani, wobei diese Lokalisierungsmittel durch den äußeren Gehörgang bedienbar sind;
- Verarbeitungsmittel (100), um ausgehend von den Koordinaten der besagten Punkte eine geradlinige Bahn (T) zu bestimmen, die durch diese zwei Punkte verläuft und einen Andockpunkt (22) auf der Außenfläche des Mastoidknochens definiert;
- wobei die Bohrmittel dazu eingerichtet sind, entlang der besagten geradlinigen Bahn zu bohren, so dass ein Durchgang bis zur Cochlea erzeugt wird.

2. Vorrichtung nach Anspruch 1, wobei die Lokalisierungsmittel einen Taster (2) mit einer empfindlichen Spitze (3) umfassen, der mit der Cochlea und dem Sinus tympani durch den äußeren Gehörgang in Kontakt gebracht werden kann, um den Eintrittspunkt und den Kontrollpunkt zu ertasten.

3. Vorrichtung nach Anspruch 2, wobei der Taster (2) am Ende eines Gelenkarms (4) montiert ist, der Positionssensoren (5) umfasst, die mit jedem seiner Freiheitsgrade assoziiert sind, so dass die Position der empfindlichen Spitze des Tasters durch die Verarbeitungseinheit von Signalen, die von den Positionssensoren erzeugt werden, abgeleitet werden kann.

4. Vorrichtung nach Anspruch 1, wobei die Bohrmittel einen motorisierten Gelenkarm umfassen, der an seinem Ende ein Bohrwerkzeug (27) trägt, wobei der besagte Arm steuerbar ist, um das Bohrwerkzeug gemäß der besagten Bahn (T) auszurichten und zu bewegen.

5. Vorrichtung nach Anspruch 3 und Anspruch 4, wobei der Taster und das Bohrwerkzeug von demselben motorisierten Gelenkarm getragen werden, der mit Positionssensoren ausgestattet ist, die mit jedem seiner Freiheitsgrade assoziiert sind.

6. Vorrichtung nach Anspruch 1, die Mittel (1) zur optischen Überwachung des äußeren Gehörgangs umfasst.

7. Vorrichtung nach Anspruch 1, die Mittel zur elektromyographischen Überwachung des Gesichtsnervs umfasst.

## Claims

1. A device for providing assistance in otologic surgery of a patient who is to be implanted with a cochlea implant, comprising drilling means (25, 27) for drilling a passage up to facing the cochlea through the mastoid bone, **characterized in that** it further comprises:
- means for identifying the positions of an entry point (21) on the surface of the cochlea and of a control point (20) in the sinus tympani, these position-identification means being operable via the external auditory canal;
- processor means (100) to determine, from coordinates of said points, a straight-line path (T) passing through those two points and defining a docking point (22) on the outside surface of the mastoid bone;
- wherein the drilling means are adapted to drill along said straight-line path, so as to create a passage up to the cochlea.

2. A device according to claim 1, wherein the position-identification means comprise a feeler (2) having a sensitive tip (3) capable of being brought into contact with the cochlea and with the sinus tympani via the external auditory canal in order to feel the entry point and the control point.

3. A device according to claim 2, wherein the feeler (2) is mounted at the end of an articulated arm (4) having position sensors (5) associated with each of its degrees of freedom, such that the position of the sensitive tip of the feeler can be deduced by the processor unit from the signals generated by the position sensors.

4. A device according to claim 1, wherein the drilling means comprise an articulated and motor-driven arm carrying a drilling tool (27) at its end, said arm being controllable to put the drilling tool into alignment with said path (T) and to move it therealong.

5. A device according to claim 3 and claim 4, wherein the feeler and the drilling tool are carried by the same arm that is articulated, motor-driven, and provided with position sensors associated with each of its degrees of freedom.

6. A device according to claim 1, including means (1) for optically monitoring the external auditory canal.

7. A device according to claim 1, including electromyographic means for monitoring the facial nerve.
